Europäisches Patentamt

European Patent Office

Office européen des brevets

(19)

(11) EP 0 645 451 B1

(12) **EUROPEAN PATENT SPECIFICATION**

(45) Date of publication and mention
of the grant of the patent:
**22.08.2001 Bulletin 2001/34**

(51) Int Cl.7: **C12N 15/18**, C07K 14/50

(21) Application number: **94114212.7**

(22) Date of filing: **09.09.1994**

(54) **Heparin binding site structural analogues of fibroblast growth factors**

Strukturanaloge von Fibroblasten-Wachstumsfaktoren, die eine modifizierte Heparin-Bindungsstelle aufweisen

Analogues structurals - par rapport au site de la liaison de héparine - de facteurs de croissance de fibroblastes

(84) Designated Contracting States:
**AT BE CH DE DK ES FR GB GR IE IT LI LU NL PT SE**
Designated Extension States:
**SI**

(30) Priority: **24.09.1993 US 126974**

(43) Date of publication of application:
**29.03.1995 Bulletin 1995/13**

(73) Proprietor: **AMERICAN CYANAMID COMPANY**
**Wayne New Jersey 07470 (US)**

(72) Inventors:
• **Li, Lu-Yuan**
  **New City, New York 10956 (US)**
• **Seddon, Andrew Peter**
  **Monroe, New York 10950 (US)**
• **Gluzman, Yakov**
  **Upper Saddle River, New Jersey 07458 (US)**
• **Bohlen, Peter**
  **Peekskill, New York 10566 (US)**

(74) Representative:
**Wächtershäuser, Günter, Prof. Dr.**
**Patentanwalt,**
**Tal 29**
**80331 München (DE)**

(56) References cited:
**EP-A- 0 298 723     EP-A- 0 363 675**
**WO-A-89/04832**

• **BIOCHEMICAL AND BIOPHYSICAL RESEARCH COMMUNICATIONS, vol. 185, no. 3, 1992 M. PRESTA et al. "Structure- -Function Relationship of Basic Fibroblast Growth Factor: Site-Directed Muta- genesis of a Putative Heparin-Binding and Receptor-Binding Region" pages 1098-1107**

**Description**

Technical Field of the Invention

This invention relates to fibroblast growth factor structural analogues, notably fibroblast growth factor-2 (formerly called basic fibroblast growth factor) analogues, which have point mutations in a heparin binding site, e.g., one or more basic residues replaced by other residues such as neutral residues. The analogues exhibit binding affinities to cell surface receptors similar to the native growth factor, but possess different heparin binding and biological properties from the native factor.

Background of the Invention

Polypeptide growth factors are hormone-like modulators of cell proliferation and differentiation. Growth factors are responsible for the regulation of a variety of physiological processes, including development, regeneration, and wound repair, and have been associated with normal as well as with pathophysiological processes. Numerous growth factors have been identified in various tissues and cells, and names that have been applied to these factors include epidermal growth factor, platelet-derived growth factor, nerve growth factor, hematopoietic growth factor, and fibroblast growth factor.

Fibroblast growth factor (FGF) was first described as an activity derived from bovine brain or pituitary tissue which was mitogenic for fibroblasts and endothelial cells. It was later noted that the primary mitogen from brain was different from that isolated from pituitary. These two factors were named acidic and basic FGF, respectively. Acidic and basic FGF are proteins containing approximately 154 amino acids. Their amino acid sequences exhibit an approximate 55% sequence homology between them.

Acidic and basic fibroblast growth factors are now known to be members of a larger family of heparin-binding growth factors which collectively trigger a variety of biological responses in many cell types, including those of mesoderm and neuroectoderm origin, such as endothelial cells, smooth muscle cells, adrenal cortex cells, prostatic and retina epithelial cells, oligodendrocytes, astrocytes, chrondocytes, myoblasts, and osteoblasts. As original family members, acidic and basic FGF are now denoted FGF-1 and FGF-2, respectively. Seven other members of the family have been identified on the basis of their modulation of cell proliferation and differentiation, and sequence homology to other FGFs.

In addition to eliciting a mitogenic response that stimulates cell growth, fibroblast growth factors can stimulate a large number of cell types to respond in a non-mitogenic manner. These activities include promotion of cell migration into wound areas (chemotaxis), initiation of new blood vessel formation (angiogenesis), modulation of nerve regeneration and survival (neurotrophism), modulation of endocrine functions, and stimulation or suppression of specific cellular protein expression, extracellular matrix production and cell survival (Baird, A., and Böhlen, P., *Handbook of Exp. Pharmacol. 95*(1): 369-418, Springer, 1990). These properties provide a basis for using fibroblast growth factors in therapeutic approaches to accelerate wound healing, nerve repair, collateral blood vessel formation, and the like. For example, fibroblast growth factors have been suggested to minimize myocardium damage in heart disease and surgery (e.g., U.S. Pat. No. 4,378,347 to Franco).

Thus, current research regarding FGF-2 and other FGFs centers on the molecular details of the receptor-mediated pathways by which the diverse physiological activities of the factors are expressed, as a way to gain information for the design of therapeutically useful agents that can either mimic or inhibit the action of the factors. The biological responses of FGF are mediated by the heparin sulfate-dependent binding of the growth factor to specific cell surface receptors, yet the molecular interactions of heparin and receptor with FGF and the exact nature of the events of the signal transduction pathway are unknown. Since the primary structure of FGF-2 isolated from a variety of sources is known, and bovine and human FGF-2 have been cloned and expressed in *E. coli* and *S. cervisiae,* recent attention has focused on the elucidation of secondary and tertiary structure and on the activity of structural analogues.

Presta and coworkers found that replacement of arginine-118, lysine-119, lysine-128, and arginine-129 of FGF-2 with glutamine by site-directed mutagenesis led to a mutant denoted by the investigators as M6B-bFGF that exhibited similar receptor binding and mitogenic activity to native growth factor, but a reduced affinity for cell surface heparin-like low affinity binding sites, a reduced chemotactic activity, and a reduced capacity to induce the production of urokinase-type plasminogen activator in cultured endothelial cells (Presta, M., *et al.*, *Biochem. Biophys. Res. Com. 185*: 1098-1107 (1992)). In addition, the mutant lacked a significant angiogenic activity *in vivo.* Replacement of lysine-128 and arginine-129 of FGF-2 by the same researchers, on the other hand, yielded a mutant denoted M6A-bFGF that exhibited different properties, i.e., a 70% inhibition of cell receptor binding (Eur. Pat. Ap. Pub. No. 363,675 to Bergonzoni, L., *et al.*).

Studies employing synthetic peptides related to the FGF sequence showed that FGF-2 (33-77) and (102-129) bind to heparin and act as weak partial agonists and antagonists in a mitogenic assay of FGF activity (Baird, A., *et al.*

*Proc. Nat. Acad. Sci. USA 85:* 2324-2328 (1988)). The same study identified a sequence, FGF-2 (115-124), involved in receptor binding. From the crystal structure of FGF-2, a cluster of basic residues appear to play a role in the binding of anionic heparin sulfate to the molecule (Zhang, *J., et al., Proc. Nat. Acad. Sci. USA 88:* 3446-3450 (1991) and Eriksson, E.A., *et al., Proc. Nat. Acad. Sci. USA 88:* 3441- 3445 (1991)). Two pairs of basic residues, lysine-128 and lysine-138 and arginine-129 and lysine-134, respectively (denoted lysine-119 and lysine-129 and arginine-120 and lysine-125 by the investigators since they employed an active truncated form of the polypeptide corresponding to residues 9-154 of the full-length sequence), were found to form ionic contacts with two ordered sulfate ions in the crystal structure (Zhang, cited above). These may mimic heparan sulfate moieties. So it is possible that the two binding sites are involved in heparin binding to FGF.

Summary of the Invention

It is an objective of the present invention to provide novel structural analogues of fibroblast growth factors, especially analogues of human fibroblast growth factor-2.

It is a further and more specific objective of the present invention to provide fibroblast growth factor structural analogues which exhibit, relative to the native factor, similar binding to cell surface receptors but different biological activities, such as decreased mitogenic activity, thereby providing fibroblast growth factor antagonists. Fibroblast growth factor antagonists are useful as potential antiangiogenesis, anticancer, antitumor, and antiproliferative agents.

These and other objects are accomplished according to the claims. The present invention provides fibroblast growth factor structural analogues which have at least one point mutation in a putative heparin binding site, i.e. replacement of a basic residue with a specific neutral residue. Some embodiments have at least one point mutation in one heparin sulfate binding site, and no mutation in another heparin binding site. Preferred analogues exhibit different heparin binding and biological activity from native growth factor; especially preferred analogues exhibit decreased affinity toward heparin and a decreased capacity to induce cell proliferation. Some embodiments exhibit a decreased angiogenesis in *in vitro* assays. Some embodiments exhibit a decreased capacity and others an increased capacity to induce the production of urokinase-type plasminogen activator. Some embodiments exhibit a decreased capacity to induce tube-like structures in cultured endothelial cells compared to native factor; others exhibit an increased capacity of the same activity. Particularly preferred analogues have at least one or two basic residues such as lysine residues replaced by glutamine.

Fibroblast growth factor-2 structural analogues of the invention include those having either lysine-138, or lysine-128 and lysine-138 replaced by glytamine residues.

The invention also provides DNA encoding the fibroblast growth factor derivatives, biologically functional circular plasmid or viral DNA vectors comprising the DNA, and procaryotic or eucaryotic host cells such as *E. coli* transformed or transfected with the vectors in a manner allowing the host cell to express the new factors.

Brief Description of the Figures

Figures 1A and 1B plot fluorescence intensities of FGF-2 (1A) and gln[128,138]FGF-2 (1B) against concentrations of heparin.

Figure 2 plots radioactivity associated with $^{125}$I-labelled FGF-2 bound to high affinity FGF-receptors on baby hamster kidney (BHK) cells against concentrations of FGF-2 and the mutants. The open circles and squares plot FGF-2 data; the closed squares plot gln[138]FGF-2 data; the closed circles plot gln[128,138]FGF-2 data; and the closed triangles plot gln[128]FGF-2 data as described hereinafter.

Figures 3A and 3B plot cell counts as a function of FGF-2 and mutant concentrations. Figure 3A shows site 1 mutants (residues lysine-128 and lysine-138), and Figure 3B shows site 2 mutants (residues arginine-129 and lysine-134).

Figure 4 shows wild-type or mutant FGF-2 (10 ng/ml) induction of urokinase-type plasminogen activator.

Figure 5 is a bar graph depicting the potency of *in vitro* angiogenic activities of wild-type or mutant FGF-2 (10 ng/ml) as measured by induction of the formation of tube-like structures by adult bovine aortic endothelial cells cultured on a type I collagen gel.

Detailed Description of the Invention

This invention is based upon the finding that replacement of certain basic residues in fibroblast growth factors with neutral residues give mutants which exhibit, in comparison to native factor, similar affinities to cell surface receptors. Some of these analogues exhibit different heparin binding and biological activities.

A protein is defined herein as a fibroblast growth factor (FGF) if it shows FGF activity in *in vitro* or *in vivo* assays and binds to heparin or heparin-like substances. By "heparin" is meant the heterogeneous, sulfated anionic polysaccharide composed of D-glucuronic acid and D-glucosamine, bound to a protein core as the "proteo-glycan" or in a free form as the "aglycan", that has potent anticoagulant properties. "Heparin-like" substances are molecules that have oligosaccharide structures related to the heparins, but may or may not have anticoagulant activity. Any type of fibroblast growth factor or mutant factor (mutein) or derivative is encompassed by this invention, particularly human fibroblast growth factor.

The fibroblast growth factor analogues of this invention are structural analogues of fibroblast growth factor having at least one basic amino acid residue replaced by another residue as defined by claim 1. Typically, the analogues have at least one one point mutation in a putative heparin binding site; many analogues have no mutation in another heparin binding site. For example, where certain basic residues are replaced by neutral residues, the analogues obtained exhibit binding affinity to baby hamster kidney (BHK) cell surface receptors similar to native growth factor. Preferred analogues exhibit binding and biological activities that are pharmacologically dissociated, so that the heparin binding and biological activity are different from the native factor. As set out in greater detail below, in many cases the biological activities exhibited by the analogues are pharmacologically dissociated. Many mutants exhibit some biological activities reduced, but others not. Preferred embodiments exhibit similar or superior binding to baby hamster kidney (BHK) cell receptors when compared to native fibroblast growth factor.

The analogues exhibit decreased affinity toward heparin from the native factor. Some embodiments also exhibit a decreased capacity to induce urokinase-type plasminogen activator production and decreased angiogenesis, exhibited as a decrease in the capacity to induce *in vitro* capillary formation. The embodiments exhibit a decreased mitogenic activity exhibited as a decreased capacity to induce cell proliferation. Other differences in the binding affinity or biological activity exhibited by the analogues in comparison to native factor are discussed hereinafter.

Structural analogues of FGF-2 are preferred in many embodiments. By "FGF-2" is meant any fibroblast growth factor-2 exhibiting a biological activity, including the 146-amino acid polypeptide originally isolated and sequenced, the 154 amino acid form currently thought to be the full polypeptide, truncated forms exhibiting activity, extended forms such as placental FGF, higher molecular weight forms described in the literature and analogues including derivatives and muteins of any of these. The term specifically includes natural FGF-2 extracted from mammalian tissue as well as recombinant polypeptides expressed from cloned DNA in *E. coli* or *S. cervisiae* from any species or expressed in insect or mammalian cells with appropriate vectors, denoted herein as "native" FGF-2.

Human FGF-2 is preferred in many embodiments. Human FGF-2 includes both native FGF-2 described above, as well as previously described structural analogues such as those having amino acid additions, amino acid substitutions, and amino acid deletions, including deletions of portions of the amino or carboxyl terminus, and chimeric proteins containing FGF at the N- or C- terminus of another protein. Example FGF-2s which can be further modified according to the invention include those having cysteine substituted with a neutral amino acid such as serine, or aspartic acid, arginine, glycine, serine, or valine substituted with other acids suggested to have enhanced stability in Eur. Pat. Ap. Pub. No. 281,822 to Seno, *et al.*; muteins formed by replacing at least one, and more preferably two, of the cysteines found in natural FGF-2 with a different amino acid residue to yield a more stable analogue (Eur. Pat. Ap. Pub. No. 320,148 to Arakawa and Fox); muteins lacking 7 to 46 amino acids from the carboxyl terminus and, optionally, having amino acid replacements suggested to have improved stability while retaining activity in Eur. Pat. Ap. Pub. No. 326,907 to Seno, *et al.*; muteins having point mutations or an N-terminal deletion suggested in Eur. Pat. Ap. Pub. No. 298,723 to Fiddes, *et al.*; the M1-bFGF to M6-bFGF muteins containing missing and substituted amino acids disclosed in Eur. Pat. Ap. Pub. No. 363,675 to Bergonzoni, cited above; readily expressed FGF prepared by replacement of Ala-3 and Ser-5 of recombinant FGF with Glu as described in Seddon, cited above and analogues thereof; analogues having an amino acid deletion, insertion or replacement in the surface loop that extends from the ninth β-strand to the tenth β-strand and having the overall secondary and tertiary structure of the original factor as described in US-A-5 491 220.

In the practice of this invention, a fibroblast growth factor derivative of this invention such as a human FGF-2 structural analogue is prepared by substituting a basic amino acid residue with another residue. Typical replacements involve the replacement of a lysine or an arginine amino acid residue with glutamine as defined by the claims.

The substitution takes place in a basic residue implicated in the binding of a sulfate moiety of heparin. Preferably, a point mutation in one putative heparin binding site occurs; in many embodiments, there is no mutation in another heparin binding site. Heparin, a highly sulfated anionic polysaccharide, protects both FGF-1 and FGF-2 from inactivation by elevated temperature, acidic conditions, and digestion from proteases, and potentiates the mitogenic activity of FGF-1. Interaction of FGF with cell surface proteoglycans has been suggested to be necessary for the binding of FGF-2 to its high affinity cell membrane receptors, and hence modulation of FGF activities.

The FGF-2 structural analogues of this invention have at least one and preferably two point mutations such as a substitution of a lysine with glutamine in the site 2 binding site for sulfates described by Zhang, cited above. Structural analogues of FGF-2 of this invention thus include FGF-2 having lysine-138 replaced by glutamine (herein denoted gln$^{138}$FGF-2), and FGF-2 having both lysine-128 and lysine-138 replaced by glutamine (herein denoted gln$^{128,138}$FGF-2).

Gln$^{128,138}$FGF-2 is one embodiment. Binding affinities of this analogue and others to cell surface receptors are similar to that of native or wild-type FGF-2, but significant changes in heparin binding and biological activities are also observed. Dissociation constants for heparin binding for gln$^{128}$FGF-2 and gln$^{138}$FGF-2, for example, are increased by about 10-fold, and that for gln$^{128,138}$FGF-2, by about 100-fold. The marked reduction in binding affinity does not appear to result from perturbation of the structural integrity of the protein since BHK receptor binding is not significantly affected.

Diminished heparin binding is, however, associated with a significant decline in several of the biological activities of FGF-2 attributable to angiogenic responses, particularly the capacity to induce the formation of capillary-like structures by cultured endothelial cells. The mitogenic activities of gln$^{128,138}$-FGF-2, for example, are about 10-fold lower than that of the wild-type protein, whereas that of gln$^{138}$FGF-2 is unchanged. In addition, the capacity of the mutant gln$^{128,138}$FGF-2 to induce urokinase-type plasminogen activator in adult bovine aortic endothelial (ABAE) cells is markedly reduced. This analogue also exhibits a diminished capacity to induce ABAE cells to form capillary-like structures in an *in vitro* angiogenesis model described more fully hereinafter. Gln$^{128}$-FGF-2, on the other hand, exhibits tube formation and urokinase-type plasminogen activator activity similar to native fibroblast growth factor.

The structural analogues of this invention may be prepared using any chemical or biochemical means known to those skilled in the art, such as preparation by assembling polypeptides from constituent amino acids or peptides and polypeptides. Alternatively, the analogues may be prepared by recombinant protein synthesis involving preparation of DNA encoding analogue FGF, insertion of that DNA into a vector, expression of the vector in host cells, and isolation of the recombinant FGF thereby produced.

DNA encoding the FGF analogues of this invention may be prepared by altering a gene of a FGF by nucleotide deletions, nucleotide additions, and/or point mutations produced using standard means. Illustrations using cassette mutagenesis are given hereinafter. Because of the degeneracy of the genetic code, a variety of codon change combinations can be selected to form DNA that encodes the FGF analogues, so that any nucleotide deletion(s), addition(s) and/or point mutations that result in a DNA encoding an analogue are encompassed by this invention. Since certain codons are more efficient for polypeptide expression in certain types of organisms, the selection of fibroblast gene alterations to yield DNA material that codes for the analogues of this invention are preferably those that yield the most efficient expression in the type of organism which is to serve as the host of the recombinant vector. Altered codon selection may also depend upon vector construction considerations.

Fibroblast growth factor DNA starting material that is altered to form DNA coding for the FGF analogues of the invention may be natural, recombinant or synthetic. Thus, DNA starting material is isolated from tissue or tissue culture, constructed from oligonucleotides using conventional methods, obtained commercially, or prepared by isolating RNA encoding FGF from fibroblasts, and using the RNA so obtained to synthesize single-stranded cDNA which is used as a template to synthesize the corresponding double stranded DNA.

Illustrating the present invention are cloned complementary DNA sequences defining human FGF-2 analogues such as that constructed in Example 1. Also encompassed are DNA sequences homologous or closely related to complementary DNA described herein, namely DNA sequences which hybridize, particularly under stringent conditions resulting in pairing only between nucleic acid fragments that having a high frequency of complementary base sequences, to FGF analogue cDNA, and RNA corresponding thereto. In addition to the FGF-encoding sequences, DNA encompassed by this invention may contain additional sequences, depending upon vector construction sequences, that facilitate expression of the gene.

DNA encoding the FGF analogues of this invention, or RNA corresponding thereto, are then inserted into a vector, e.g., a pBR, pUC, pUB or pET series plasmid, and the recombinant vector used to transform microbial host organisms. Host organisms useful in the invention are bacterial (e.g., *E. coil or B. subtilis),* yeast (e.g., *S. cervisiae*), mammalian (e.g., mouse fibroblast), or insect cells. This invention thus also provides novel, biologically functional viral and circular plasmid RNA and DNA vectors incorporating RNA and DNA sequences describing the FGF analogues generated. Culture of host organisms stably transformed or transfected with such vectors under conditions facilitative of large scale expression of the exogenous, vector-borne DNA or RNA sequences and isolation of the desired polypeptides from the growth medium, cellular lysates, or cellular membrane fractions yields the desired products. An example of expression of FGF-2 analogues in *E. coil* is given in Example 1.

The present invention provides for the total and/or partial manufacture of DNA sequences coding for FGF-2 analogues, and including such advantageous characteristics as incorporation of codons preferred for expression by selected non-mammalian hosts, provision of sites of cleavage by restriction by endonuclease enzymes, and provision of additional initial, terminal or intermediate DNA sequences which facilitate construction of readily expressed vectors. Correspondingly, the present invention provides for manufacture (and development by site specific mutagenesis of cDNA and genomic DNA) of DNA sequences coding for microbial expression of FGF analogues which differ from the forms specifically described herein in terms of identity or location of one or more amino acid residues (i.e., deletion analogues containing less than all of the residues specified for human FGF-2, and/or substitution analogues wherein one or more residues are added to a terminal or medial portion of the polypeptide), and which share the biological properties of FGF-2 analogues described herein.

DNA (and RNA) sequences of this invention code for all sequences useful in securing expression in procaryotic or eucaryotic host cells of polypeptide products having at least a part of the primary structural conformation, and one or more of the biological properties of FGF analogues which are comprehended by: (a) the DNA sequences encoding FGF-2 analogues as described herein, or complementary strands; (b) DNA sequences which hybridize to DNA sequences defined in (a) or fragments thereof; and (c) DNA sequences which, but for the degeneracy of the genetic code, would hybridize to the DNA sequences defined in (a) and (b) above. Specifically comprehended are genomic DNA sequences encoding allelic variant forms of FGF analogues included therein, and sequences encoding analogue RNA, fragments thereof, and RNA or DNA sequences that incorporate codons facilitating transcription or RNA replication of messenger RNA in non-vertebrate hosts.

Isolation and purification of microbially expressed polypeptides provided by the invention are by conventional means including, for example, preparative chromatographic separations such as that illustrated in Example 1 utilizing heparin, and immunological separations, including monoclonal and/or polyclonal antibody preparations.

A mutation involving Lys-138 exhibits decreased heparin binding, but no significant change in endothelial cell proliferation compared to native factor. These analogues may thus be used as FGF-2 agonists. By "agonist" is meant any substance that has affinity for and stimulates physiological activity at cell receptors normally stimulated by naturally occurring substances. The FGF analogues that are agonists of FGF activity can promote vascularization, cell growth, and/or cell survival, and thus have application in tissue repair such as healing of wounds, burns, bone fractures, surgical abrasions, ulcers, and the like as well as tissue repair during ischemia and myocardial infarction.

In contrast, as summarized above and described in more detail in the examples below, mutations involving Arg-128 and Lys-138 such as $gln^{128,138}$FGF-2 exhibit reduced heparin binding, reduced mitogenic activity, reduced capacity to induce urokinase-type plasminogen activator, and reduced angiogenesis *in vitro.* Thus, reduced heparin binding seems cause a deficiency in the signal transducing pathways which exert FGF-2 activities, such as induction of cell proliferation, urokinase production, and *in vitro* capillary formation. As such, the analogue is expected to exhibit an antagonist activity.

By "antagonist" is meant any substance that tends to nullify the action of another, as one, for example, that binds to a cell receptor without eliciting a biological response. For FGFs, antagonists include, but are not limited to, substances which bind to FGF receptors but do not stimulate proliferation or migration of fibroblasts, endothelial cells and astroglial cells. FGF antagonists are useful in the treatment of certain neoplasms, particularly angiogenic neoplasms, as a valuable tool for inhibiting tumoral angiogenesis as well as an anticancer and antiproliferative agent. The antagonists additionally act as angiogenesis inhibitors and, therefore, are useful for the treatment of diseases where neovascularization is dominant in the pathology, such as retinopathies of the eye, neovascular glaucoma, skin disorders, chronic inflammation, rheumatoid arthritis, and the like. As used herein, the terms agonist and antagonist are not limiting, and a single factor may exhibit antagonism to one biological function mediated by native factor, and agonism to another function. Where this occurs, the biological activities of native factor are said to be pharmacologically dissociated in the analogue.

The following examples are presented to further illustrate and explain the present invention and should not be taken as limiting in any regard.

Example 1

Human FGF-2 analogues are constructed, expressed and purified.

A gene encoding human $glu^{3,5}$FGF-2 is first prepared as described in Seddon, A.P., *et al*., *Annals N.Y. Acad. Sci. 638:* 98-108 (1991). Briefly stated, a synthetic gene encoding the 155 amino acid form of human FGF-2 cloned into pUC 18 is purchased from British Bio-technology, Oxford, UK. The nucleotide sequence (2-49) to be changed is excised from pUC 18 with restriction endonucleases *Hind*III and *Bsp*MII and a synthetic fragment encoding the first 5 N-terminal amino acids of FGF-1 and containing an internal *Nde*1 site is cloned into pUC 18, yielding a construct encoding glutamic acid at positions 3 and 5. The cDNA encoding FGF-2 is then excised from pUC 18 with *Nde*1 and *Bam*H1 and cloned into the *Nde*1 and *Bam*H1
site of the expression vector pET-(M13) ΔPS, a derivative of pET-3a (Rosenberg, A.H., *et al*., *Gene 56*: 125-135 (1987)).

The plasmid is modified by site-directed mutagenesis using single-stranded DNA and two primers, 5'-GTCTAGAAAA TACACCAGTT GGTACGTAGC ACTGCAGCGA ACCGGTCAGT ATAAA-3' (SEQ ID NO 1) and 5'-CTTGGTTCCC AAACAGGGCC CGGGCAGAAA GCTA-3' (SEQ ID NO 2) to obtain the $gln^{128}$-FGF-2 mutant gene, that contains newly introduced unique restriction sites *Sna*B1, *Pst*1 and *Apa*1 (underlined), respectively. These flanking restriction sites in the plasmid are digested with the cognate restriction enzymes so that short synthetic duplex DNA cassettes can be inserted using cassette-directed mutagenesis as described by Wells, *et al. (Gene 34:* 315-323 (1985)).

Cassette directed mutagenesis of this variant gene using *Sna*B1 and *Apa*1 and two sets of annealed oligonucleotides 5'-GTAGCACTTA AGCGTACGGG GCAGTATAAG CTTGGTTCCC AAACAGGGCC-3' (SEQ ID NO 3) and 5'-CTGTTTGGGA ACCAAGCTTA TACTGCCCCG TACGCTTAAG TGCTAC-3' (SEQ ID NO 4), and 5'-GTAGCACTGC AGCGTACGGG GCAGTATAAG CTTGGTTCCC AAACAGGGCC-3' (SEQ ID NO 5) and 5'-CTGTTTGGGA ACCAAGCTTA TACTGCCCCG TACGCTGCAG TGCTAC-3' (SEQ ID NO 6) gives, respectively, a mutant gln[138]FGF-2 that has new restriction sites *Af12* and *Hind3* (underlined), and a double mutant gln[128,138]-FGF-2 that has new restriction site *Hind*3 (underlined).

The mutants are then expressed and purified. The plasmids containing the genes encoding the seven mutants described above are transformed into competent *E. coli* BL21 plys S and cultured at 37°C in Luria broth containing 50 ug/ml ampicillin and 30 ug/ml chloramphenicol until absorbance at 600 nm of 0.4 is reached. Expression of the recombinant protein is induced by the addition of 2 mM isopropylthiogalactoside for 2 hours at 37°C. Cells from a 1 liter culture are harvested by centrifugation, resuspended in 30 ml of 50 mM tris-HCl, pH 7.5, containing 0.1 mM EDTA and 0.6 M NaCl, and disrupted by treatment with lysozyme (10 ug/ml) for 20 minutes at 4°C followed by sonication (6 x 30 sec pulses). The lysates are clarified by centrifugation (10,000 x g, 20 minutes), and the supernatant solutions incubated with 5 ml of hydrated heparin Sepaharose (Pharmacia/LKB) at 4°C for 1 hour with constant rotation. The resin is isolated by filtration on 0.8 *u*m filter apparatus (Nalgene), washed extensively with 10 mM Tris-HCl pH 7.4, containing 0.6 M NaCl, and bound protein is eluted with Tris buffer containing 3 M NaCl (25 ml). The 3 M NaCl eluent is diluted 6-fold with Tris buffer and loaded onto a TSK heparin 5PW column (0.21 x 15 cm; the Nest Group, MA) and the column developed using a linear gradient (0.6-2 M NaCl) in 90 minutes at a flow rate of 3 ml/minute.

*E. coli* (BL21 plys S) strains harboring the mutants were deposited in the American Type Culture Collection (A.T.C.C.). A strain harboring gln[138]-FGF-2 bears A.T.C.C. accession number 69420, and a strain harboring gln[128,138]-FGF-2 bears A.T.C.C. accession number 69418.

Example 2

Thermodynamic dissociation constants for heparin binding and the stoichiometry of heparin binding to the FGF-2' analogues of Example 1 are obtained.

FGF-2 contains a single tryptophan residue at position 123. Crystallographic data suggest that this tryptophan residue is located close to a cluster of basic residues implicated to be involved in heparin binding (Zhang, cited above), suggesting that the fluorescence emission of the tryptophan residue may be used to report changes in protein conformation upon the binding of various ligands. Therefore, the fluorescence properties of FGF-2 and FGF-2 mutants in the presence and absence of heparin and a heparin mimic, β-cyclodextran tetradecasulfate (BCDS) are evaluated in this example.

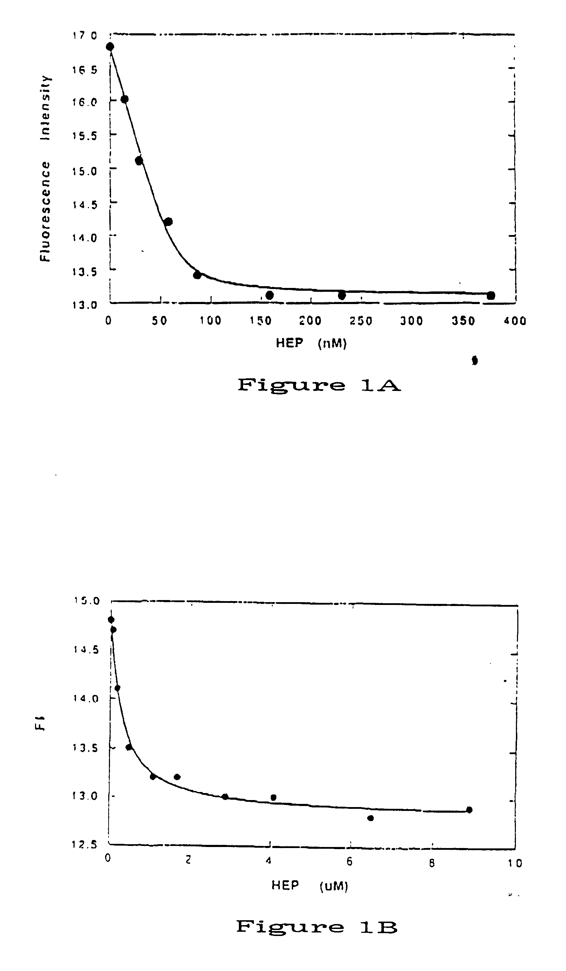

Fluorescence titration measurements are conducted in a Perkin-Elmer LS-5 fluorospectrometer using excitation and emission wavelengths of 290 nm and 350 nm, respectively. Two milliliters of FGF solution in 10 mM Tris-HCl, pH 7.4, containing 2 mM of dithiothreitol are placed in a cuvette. A small aliquot (~2-5 *u*l) of heparin (Hepar Industries, Franklin, OH) or BCDS (American Maize-Products, Hammand, IN) is added to the cuvette each time before the fluorescence intensity of the solution is read against a solution of tryptophan that gives comparable fluorescence intensity and to which an identical amount of heparin or BCDS is added each time. Concentrations of FGF-2 and gln[128,138]FGF-2 are 370 nM and 280 nM, respectively. The results are plotted in Figures 1A and 1B, and show that tryptophan fluorescence emission is quenched upon addition of heparin to the protein solution. An equivalent tryptophan residue, Trp122, exists in acidic FGF (FGF-1), for which similar fluorescence quenching is obtained. The addition of BCDS to the protein solution also results in quenching of fluorescence of FGF-2, whereas the addition of the nonsulfated compound (BCD) shows no spectral effects, indicating that sulfation of the ligand is required for binding and change in fluorescence properties of the protein.

The thermodynamic dissociation constant ($K_D$) is obtained from plots of fluorescence intensity versus ligand concentration. The fluorescence intensity data are fitted by non-linear regression by using equation 1 below, where $F_O$ is observed fluorescence intensity, $F_I$ is initial intensity, $\Delta F$ is the difference between final and the initial fluorescence intensities, $C_t$ is the total FGF protein concentration, $K_D$ is the thermodynamic dissociation constant, and $L_t$ is the total heparin concentration. It is assumed in equation 1 that the stoichiometry of FGF and heparin in the complex is 1:1. A graphic software Kaleidagraph is used for handling data.

$$(1) \qquad F_0 = F_I + \frac{\Delta F((C_t + K_D + L_t) - \sqrt{(C_t + K_D + L_t)^2 - 4C_tL_t}}{2C_t}$$

However, in the fluorescence titration experiments the titration curves shown in Figure 1 cannot be fitted directly using equation 1. This indicates that the stoichiometry of the protein-ligand complex is not equal to unity. Since the heparin preparation used here is composed of an average of 20 sulfated monosaccharide units per chain, it is likely that more than one FGF molecule is bound per oligosaccharide chain. The extensively positive charges on the surface of the protein and the strong negative charges of the ligands suggest that ionic interactions appear to be the main driving force in the binding of heparin to FGF. Moreover, it is reasonable to assume that, in the absence of any data to indicate otherwise, the binding sites on heparin are independent and the binding affinities are similar. By the same token, BCDS is a seven-membered cyclic oligosaccharide with 14 negatively charged sulfate groups and could possess several binding sites for FGF. Under this assumption, the effective concentration of the ligand needs to be adjusted by the multiplication of the actual concentrations by the number of possible binding sites. If n is the average number of FGF molecules bound per oligosaccharide, then equation 1 can be modified to give

$$(2) \qquad F_0 = F_I + \frac{\Delta F((C_t + K_D + nL_t) - \sqrt{(C_t + K_D + nL_t)^2 - 4nC_tL_t}}{2C_t}$$

in which the effective concentration of the ligand is increased by n times.

Equation 2 is used to fit the data shown in Figure 1 and the values of the two variables, $K_D$ and n, are determined by non-linear regression of at least three titration curves for each molecule. An average of four (n = 4) FGF-2 molecules interacting with heparin is obtained. Four binding sites on each heparin molecule are assumed for mutant gln[128,138]FGF-2. Since the molecular weight of heparin used in the experiments is about 5000, which has an average of 20 monosaccharide units, about 4 to 6 monosaccharide units may interact with each FGF molecule. Two to four FGF molecules can bind to one BCDS (which has a calculated molecular weight of 2226). This estimate is in good agreement with that reported by Mach, *et al.* (*Biochemistry* 32: 5480-5489 (1993)) of a stoichiometry of 1:4-6 determined by a variety of different approaches including light scattering and analytical ultracentrifugation analyses.

Table I presents the thermodynamic dissociation constants obtained from fluorescence quenching experiments for the wild-type FGF and the mutants using heparin or BCDS.

Table I

| Thermodynamic dissociation constants ($K_D$) associated with the binding of heparin to FGF-2 and the mutants. | | |
|---|---|---|
| *FGF* | *$K_D$ Heparin (nM)* | *$K_D$ BCDS (nM)* |
| FGF-1 | $2 \pm 0.2$ | $4 \pm 2$ |
| FGF-2 | $14 \pm 6$ | $14 \pm 7$ |
| gln[138]FGF-2 | $126 \pm 53$ | $228 \pm 23$ |
| gln[128,138]FGF-2 | $1280 \pm 380$ | $2700 \pm 600$ |

It can be seen from the data that the $K_D$ values change dramatically for site 1 mutants but not for the proteins containing mutations at site 2. The affinity toward heparin decreases about 10-fold for mutant gln[138]FGF-2 and about 100-fold for the double mutant gln[128,138]FGF-2. Similar results are observed with BCDS.

Example 3

Binding and relevant biological activity studies using the FGF-2 mutants isolated and purified in Example 1 are made.

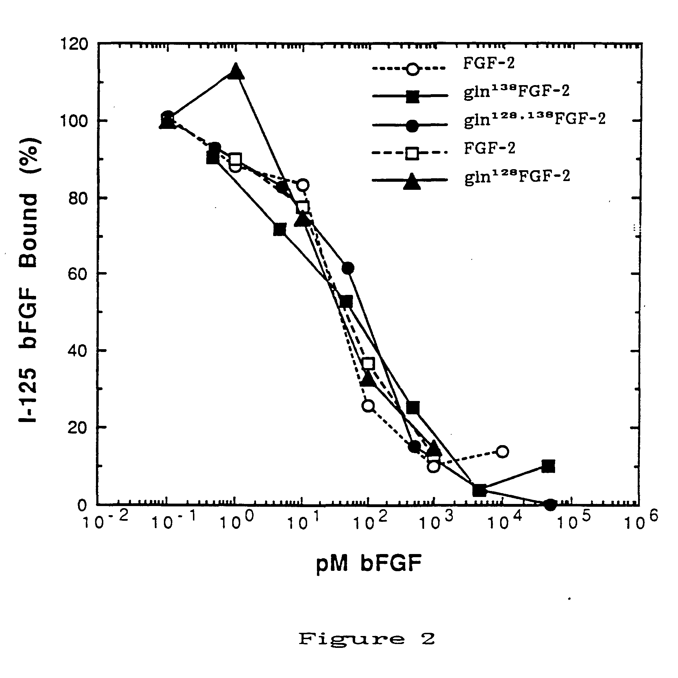

The mutant proteins are tested for their capacity to compete for the binding of $^{125}$I-FGF-2 (Amersham Corp.) to baby hamster kidney (BHK) cells, which express high numbers of FGF receptors, as described by Moscatelli (*J. Cell Physiol. 131*: 123-130 (1987)). Briefly, cells plated on 24-well plates are incubated with 50 pM $^{125}$I-FGF-2 with serial dilutions of unlabelled FGF-2 or the mutants at room temperature for 1 hour. The cells are then incubated at 4°C for 30 minutes, washed twice with phosphate-buffered saline and treated with 20 mM Hepes, pH 7.5, containing 2 M NaCl to remove $^{125}$I-FGF-2 bound to low affinity heparan sulfate binding sites. Receptor-bound FGF-2 is recovered by treatment of the cells with 0.5% Triton X-100 in 0.1 M sodium phosphate, pH 8, and counted in a gamma-counter. Assays are conducted in duplicate. The results are plotted in Figure 2. No significant difference is observed for any of the FGF-2 mutants as compared to the wild type.

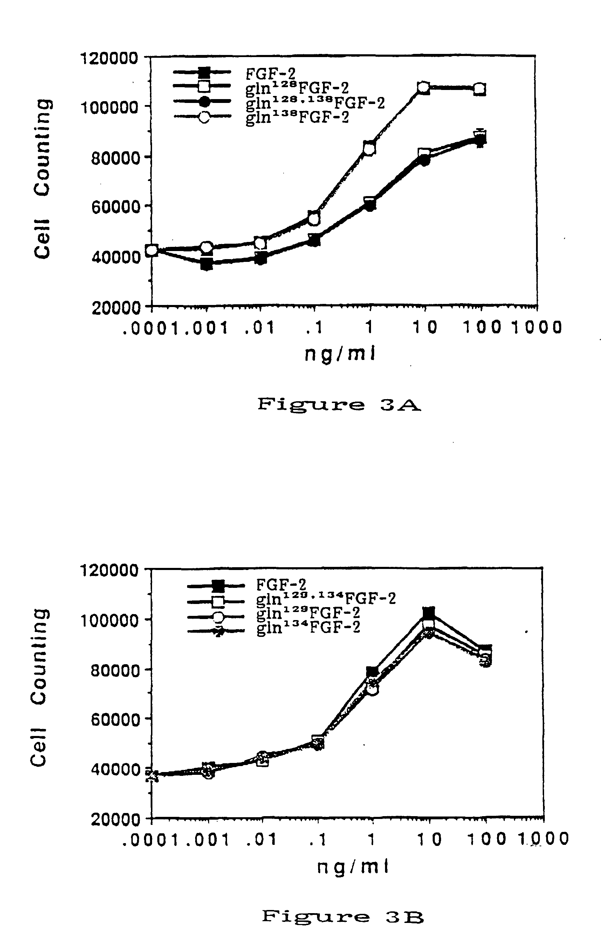

The mitogenic activity of FGF-2 and mutants are determined using bovine vascular endothelial cells derived from adult aortic arch as described by Gospardarowicz, *et al.* (*Proc. Nat. Acad. Sci. 81*: 6963-6967 (1984)). Briefly, cells are seeded at an initial density of 8000 cells per 24-well plate in 0.5 ml Dulbecco's modified Eagle's medium (DMEM) containing 10% calf serum (Hyclone, Logan, UT) supplemented with penicillin (100 units/ml), streptomycin (100 *u*g/ml) and L-glutamine (2 mM). Two hours after plating, 20 ul aliquots of serial dilutions of FGF-2 and mutants in DMEM are added. After 5 days in culture, duplicate plates are trypsinized and cell densities determined by cell counting in a Coulter counter. Determinations are conducted in duplicate. The results are plotted in Figure 3.

The activity of FGF-2 and the mutants to induce proliferation of ABAE cells is determined as a function of added FGF concentrations in the culture media. The $EC_{50}$ values for mutant $gln^{128,138}$FGF-2 in the site 1 group (Figure 3A) show about a 10-fold increase, from 0.5 ng/ml to 5.0 ng/ml, whereas mutant $gln^{138}$FGF-2 and all site-2 mutants show no significant change as compared to the wild-type value (Figure 3B).

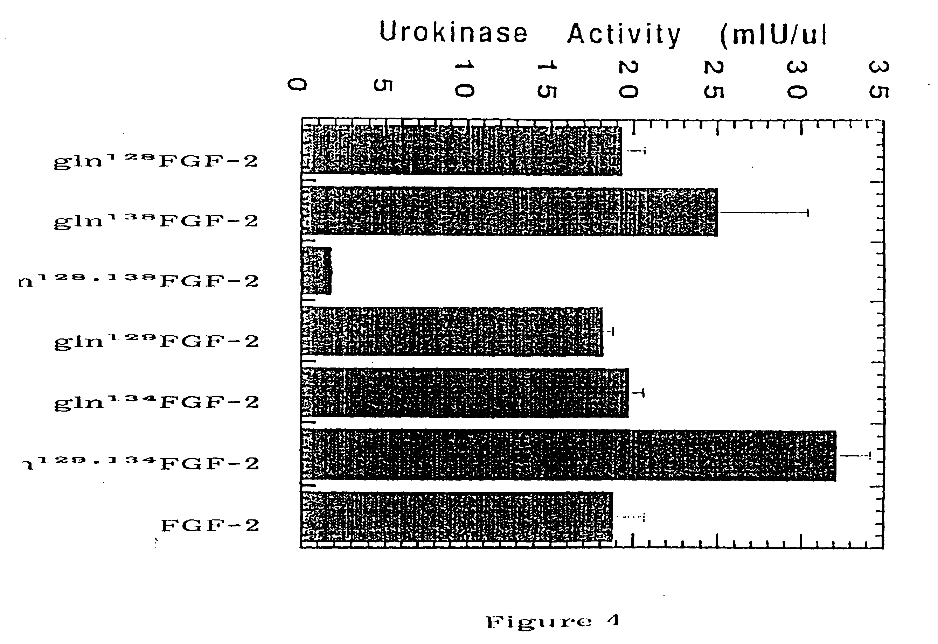

Urokinase-type plasminogen activator induction by the FGF mutants is evaluated. Adult bovine aortic endothelial (ABAE) cells are seeded at 20,000 cells/well in 96-well plates and maintained in DMEM containing 10% calf serum (Hyclone, Logan, UT) supplemented with penicillin (100 units/ml), streptomycin (100 $u$g/ml) and L-glutamine (2 mM) and different concentrations of FGF-2 or mutants. After 24 hours, the cells are washed with phosphate-buffered saline and lysed in 60 mM Tris-HCl, pH 8.5, containing 0.05% Triton X-100. Cell-associated urokinase-type plasminogen activator (uPA) activity is measured as described by Presta, *et al.*, cited above, using the plasmin chromogenic substrate D-norleucyl-hexahydrotyrosyl-lysine p-nitroanilide acetate (American Diagnostics, Greenwich, CT). Cell-associated protein concentrations are determined using Coomassie blue binding to protein. The results are plotted in Figure 4. Mutant $gln^{128,138}$FGF-2 is the only mutant that exhibits a markedly reduced capability to induce uPA.

*In vitro* angiogenesis evaluations are made by observing whether the mutants induce capillary-like structures in ABAE cells cultured on a 3-dimensional collagen gel. Three-dimensional collagen gel plates (24-well) are prepared by adding 0.5 ml chilled solution of 0.7 mg/ml rat-tail type I collagen (Becton Dickinson Labwares, Bedford, MA) containing 1X DMEM and adjusting to neutral pH with $NaHCO_3$ to each well. After formation of collagen gel (about 1-2 mm thickness), ABAE cells are seeded at 50,000 cells/well. The cultures are maintained at 37°C in DMEM containing 10% calf serum (Hyclone, Logan, UT) supplemented with penicillin (100 units/ml), streptomycin (100 $u$g/ml) and L-glutamine (2 mM) until the cultures reach confluency, usually in 5 days by which time the cells form a monolayer on the gel. The medium is then replaced with fresh medium containing different concentrations of FGF-2 or mutants. The cultures are maintained at 37°C for 48 hours, then discontinued by fixation with cold methanol (-20°C).

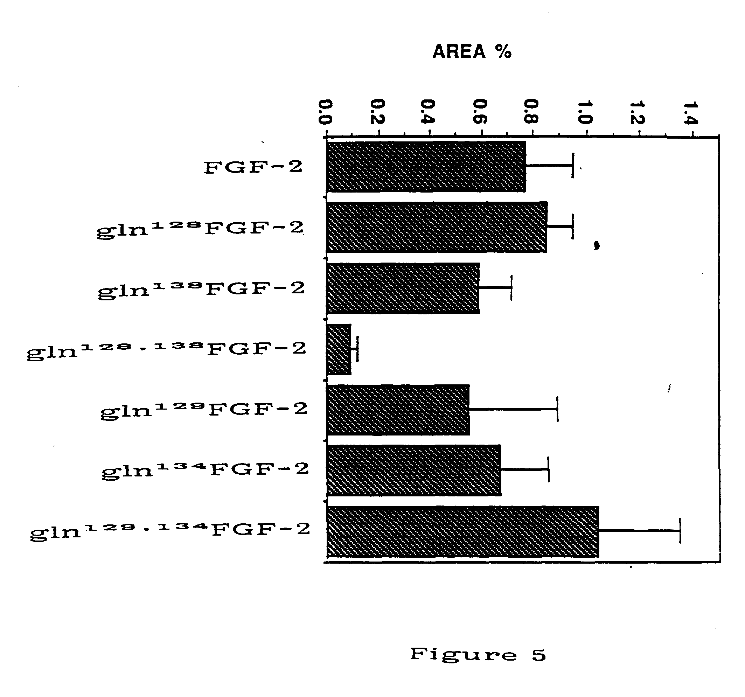

The abundance of the capillary-like structures formed by ABAE cells is analyzed by using a Kontron IBAS Image Analyzer assisted with a Hamamatsu C2400 video camera and a Zeiss Axioshop microscope. The phase-contrast image of each field obtained with a video camera is converted to a binary image in which the areas occupied by capillary-like structures are white and the rest is a black background. The extent of *in vitro* angiogenesis is then measured as a percentage of the white areas divided by the total area measured. Cell cultures of identical conditions are maintained in duplicate wells. Three to four areas of each well are examined by image analysis and the mean value and standard deviation are determined. The results from this computer-assisted quantitation method are set out in Figure 5. Tube formation induced by mutant $gln^{128,138}$FGF-2 is much less than that indicated by wild-type FGF-2 and other mutants.

The above description is for the purpose of teaching the person of ordinary skill in the art how to practice the present invention, and it is not intended to detail all those obvious modifications and variations of it which will become apparent to the skilled worker upon reading the description. It is intended, however, that all such obvious modifications and variations be included within the scope of the present invention as defined in the appended claims.

SEQUENCE LISTING

(1) GENERAL INFORMATION

(i) APPLICANTS:

Lu-Yuan Li
Andrew P. Seddon
Yakov Gluzman
Peter Bohlen

(ii) TITLE OF INVENTION: Heparin Binding Site Structural Analogues of Fibroblast Growth Factors

(iii) NUMBER OF SEQUENCES: 14

(iv) CORRESPONDENCE ADDRESS:

American Cyanamid Company
Patent Law Department
One Cyanamid Plaza
Wayne, NJ 07470-8426

(v) COMPUTER READABLE FORM

    (A) MEDIUM TYPE: 5.25" 360 Kb diskette
    (B) COMPUTER: IBM PC
    (C) OPERATING SYSTEM: MS DOS
    (D) SOFTWARE: Word Processor

(viii) ATTORNEY INFORMATION

    (A) NAME: Estelle J. Tsevdos
    (B) REGISTRATION NUMBER: 31145
    (C) DOCKET NUMBER: 854-011

(ix) TELECOMMUNICATION INFORMATION

    (A) TELEPHONE NUMBER: 201-831-3242
    (B) TELEFAX NUMBER: 201-831-3305

(2) INFORMATION FOR SEQ ID NO: 1

    (i) SEQUENCE CHARACTERISTICS

        (A) LENGTH: 55 bases
        (B) TYPE: nucleic acid
        (C) STRANDEDNESS: single
        (D) TOPOLOGY: linear

    (ii) MOLECULE TYPE
        (A) DESCRIPTION: oligonucleotide
    (v) FRAGMENT TYPE: synthetic DNA
    (ix) FEATURE
        (D) INFORMATION: primer used in preparing constructs
    (xi) SEQUENCE DESCRIPTION: SEQ ID NO 1:

**GTCTAGAAAA TACACCAGTT GGTACGTAGC ACTGCAGCGA ACCGGTCAGT ATAAA**

(3) INFORMATION FOR SEQ ID NO: 2

    (i) SEQUENCE CHARACTERISTICS

        (A) LENGTH: 34 bases
        (B) TYPE: nucleic acid
        (C) STRANDEDNESS: single
        (D) TOPOLOGY: linear

    (ii) MOLECULE TYPE
        (A) DESCRIPTION: oligonucleotide
    (v) FRAGMENT TYPE: synthetic DNA
    (ix) FEATURE
        (D) INFORMATION: primer used in preparing constructs
    (xi) SEQUENCE DESCRIPTION: SEQ ID NO 2:

**CTTGGTTCCC AAACAGGGCC CGGGCAGAAA GCTA**

(4) INFORMATION FOR SEQ ID NO: 3

(i) SEQUENCE CHARACTERISTICS

    (A) LENGTH: 50 bases
    (B) TYPE: nucleic acid
    (C) STRANDEDNESS: single
    (D) TOPOLOGY: linear

(ii) MOLECULE TYPE
    (A) DESCRIPTION: oligonucleotide
(v) FRAGMENT TYPE: synthetic DNA
(ix) FEATURE
    (D) INFORMATION: used in preparing constructs annealed with SEQ ID NO 4
(xi) SEQUENCE DESCRIPTION: SEQ ID NO 3:

**GTAGCACTTA AGCGTACGGG GCAGTATAAG CTTGGTTCCC AAACAGGGCC**

(5) INFORMATION FOR SEQ ID NO: 4

(i) SEQUENCE CHARACTERISTICS

    (A) LENGTH: 46 bases
    (B) TYPE: nucleic acid
    (C) STRANDEDNESS: single
    (D) TOPOLOGY: linear

(ii) MOLECULE TYPE
    (A) DESCRIPTION: oligonucleotide
(v) FRAGMENT TYPE: synthetic DNA
(ix) FEATURE
    (D) INFORMATION: used in preparing constructs annealed with SEQ ID NO 3
(xi) SEQUENCE DESCRIPTION: SEQ ID NO 4:

**CTGTTTGGGA ACCAAGCTTA TACTGCCCCG TACGCTTAAG TGCTAC**

(6) INFORMATION FOR SEQ ID NO: 5

(i) SEQUENCE CHARACTERISTICS

    (A) LENGTH: 50 bases
    (B) TYPE: nucleic acid
    (C) STRANDEDNESS: single
    (D) TOPOLOGY: linear

(ii) MOLECULE TYPE
    (A) DESCRIPTION: oligonucleotide
(v) FRAGMENT TYPE: synthetic DNA
(ix) FEATURE
    (D) INFORMATION: used in preparing constructs annealed with SEQ ID NO 6
(xi) SEQUENCE DESCRIPTION: SEQ ID NO 5:

**GTAGCACTGC AGCGTACGGG GCAGTATAAG CTTGGTTCCC AAACAGGGCC**

(7) INFORMATION FOR SEQ ID NO: 6

(i) SEQUENCE CHARACTERISTICS

(A) LENGTH: 46 bases
(B) TYPE: nucleic acid
(C) STRANDEDNESS: single
(D) TOPOLOGY: linear

(ii) MOLECULE TYPE
     (A) DESCRIPTION: oligonucleotide
(v) FRAGMENT TYPE: synthetic DNA
(ix) FEATURE
     (D) INFORMATION: used in preparing constructs annealed with SEQ ID NO 5
(xi) SEQUENCE DESCRIPTION: SEQ ID NO 6:


**CTGTTTGGGA ACCAAGCTTA TACTGCCCCG TACGCTGCAG TGCTAC**


(8) INFORMATION FOR SEQ ID NO: 7

(i) SEQUENCE CHARACTERISTICS

(A) LENGTH: 28 bases
(B) TYPE: nucleic acid
(C) STRANDEDNESS: single
(D) TOPOLOGY: linear

(ii) MOLECULE TYPE
     (A) DESCRIPTION: oligonucleotide
(v) FRAGMENT TYPE: synthetic DNA
(ix) FEATURE
     (D) INFORMATION: used in preparing constructs annealed with SEQ ID NO 8
(xi) SEQUENCE DESCRIPTION: SEQ ID NO 7:


**GTAGCACTGC AGCAGACGGG GCAGTATC**


(9) INFORMATION FOR SEQ ID NO: 8

(i) SEQUENCE CHARACTERISTICS

(A) LENGTH: 32 bases
(B) TYPE: nucleic acid
(C) STRANDEDNESS: single
(D) TOPOLOGY: linear

(ii) MOLECULE TYPE
     (A) DESCRIPTION: oligonucleotide
(v) FRAGMENT TYPE: synthetic DNA
(ix) FEATURE
     (D) INFORMATION: used in preparing constructs annealed with SEQ ID NO 7
(xi) SEQUENCE DESCRIPTION: SEQ ID NO 8:


**AGCTGATACT GCCCCGTCTG CTGCAGTGCT AC**

(10) INFORMATION FOR SEQ ID NO: 9

(i) SEQUENCE CHARACTERISTICS

(A) LENGTH: 50 bases
(B) TYPE: nucleic acid
(C) STRANDEDNESS: single
(D) TOPOLOGY: linear

(ii) MOLECULE TYPE
   (A) DESCRIPTION: oligonucleotide
(v) FRAGMENT TYPE: synthetic DNA
(ix) FEATURE
   (D) INFORMATION: used in preparing constructs annealed with SEQ ID NO 10
(xi) SEQUENCE DESCRIPTION: SEQ ID NO 9:

**GTAGCACTTA AGCAGACGGG GCAGTATCAG CTTGGTTCCA AAACAGGGCC**

(11) INFORMATION FOR SEQ ID NO: 10

(i) SEQUENCE CHARACTERISTICS

(A) LENGTH: 46 bases
(B) TYPE: nucleic acid
(C) STRANDEDNESS: single
(D) TOPOLOGY: linear

(ii) MOLECULE TYPE
   (A) DESCRIPTION: oligonucleotide
(v) FRAGMENT TYPE: synthetic DNA
(ix) FEATURE
   (D) INFORMATION: used in preparing constructs annealed with SEQ ID NO 9
(xi) SEQUENCE DESCRIPTION: SEQ ID NO 10:

**CTGTTTTGGA ACCAAGCTGA TACTGCCCCG TCTGCTTAAG TGCTAC**

(12) INFORMATION FOR SEQ ID NO: 11

(i) SEQUENCE CHARACTERISTICS

(A) LENGTH: 43 bases
(B) TYPE: nucleic acid
(C) STRANDEDNESS: single
(D) TOPOLOGY: linear

(ii) MOLECULE TYPE
   (A) DESCRIPTION: oligonucleotide
(v) FRAGMENT TYPE: synthetic DNA
(ix) FEATURE
   (D) INFORMATION: used in preparing constructs annealed with SEQ ID NO 12
(xi) SEQUENCE DESCRIPTION: SEQ ID NO 11:

**TTAAGCAGAC GGGGCAGTAT AAGCTTGGTT CCAAAACAGG GCC**

(13) INFORMATION FOR SEQ ID NO: 12

(i) SEQUENCE CHARACTERISTICS

(A) LENGTH: 35 bases

(B) TYPE: nucleic acid
(C) STRANDEDNESS: single
(D) TOPOLOGY: linear

(ii) MOLECULE TYPE
    (A) DESCRIPTION: oligonucleotide
(v) FRAGMENT TYPE: synthetic DNA
(ix) FEATURE
    (D) INFORMATION: used in preparing constructs annealed with SEQ ID NO 11
(xi) SEQUENCE DESCRIPTION: SEQ ID NO 12:


CTGTTTTGGA ACCAAGCTTA TACTGCCCCG TCTGC


(14) INFORMATION FOR SEQ ID NO: 13

(i) SEQUENCE CHARACTERISTICS

    (A) LENGTH: 43 bases
    (B) TYPE: nucleic acid
    (C) STRANDEDNESS: single
    (D) TOPOLOGY: linear

(ii) MOLECULE TYPE
    (A) DESCRIPTION: oligonucleotide
(v) FRAGMENT TYPE: synthetic DNA
(ix) FEATURE
    (D) INFORMATION: used in preparing constructs
(xi) SEQUENCE DESCRIPTION: SEQ ID NO 13:


TTAAGCGTAC GGGGCAGTAT CAGCTTGGTT CCAAAACAGG GCC


(15) INFORMATION FOR SEQ ID NO: 14

(i) SEQUENCE CHARACTERISTICS

    (A) LENGTH: 34 bases
    (B) TYPE: nucleic acid
    (C) STRANDEDNESS: single
    (D) TOPOLOGY: linear

(ii) MOLECULE TYPE
    (A) DESCRIPTION: oligonucleotide
(v) FRAGMENT TYPE: synthetic DNA
(ix) FEATURE
    (D) INFORMATION: used in preparing constructs
(xi) SEQUENCE DESCRIPTION: SEQ ID NO 14:


CTGTTTTGGA ACCAAGCTGAT ACTGCCCCGT ACGC


BIBLIOGRAPHY

Arakawa, T. and Fox G.M., *Eur. Pat. Ap. No.* 320,148 (1989).
Baird, A., and Böhlen, P., *Handbook of Exp. Pharmacol.* 95(1): 369-418, Springer, 1990.
Baird, A., *et al., Proc. Nat. Acad. Sci. USA 85:* 2324-2328 (1988).
Bergonzoni, L., *et al., Eur. Pat.* Ap. Pub. No. 363,675, 18 April 1990.

**EP 0 645 451 B1**

Eriksson, E.A., *et al., Proc. Nat. Acad. Sci. USA 88:* 3441-3445 (1991).

Fiddes, J.C., *et al.*, Eur. Pat. Ap. Pub. No. 298,723 (1989).

Franco, W.P., U.S. Pat. No. 4,378,347, Mar. 29, 1983.

Gospardarowicz, D., *et al., Proc. Nat. Acad. Sci. 81:* 6963-6967 (1984).

Mach, H., *et al., Biochemistry 32:* 5480-5489 (1993).

Moscatelli, D., *J. Cell. Physiol. 131:* 123-130 (1987).

Presta, M., *et al., Biochem. Biophys. Res. Commun. 185:* 1098-1107 (1992).

Rosenberg, A.H., *et al., Gene 56:* 125-135 (1987).

Seddon, A.P., *et al., Annals N.Y. Acad. Sci. 638:* 98-108 (1991).

Seno, M., *et al.,* Eur. Pat. Ap. Pub. Nos. 281,822 (1988) and 326,907 (1989).

Wells, J.A., *et al.*, *Gene* 34: 315-323 (1985).

Zhang, J., *et al., Proc. Nat. Acad. Sci. USA 88:* 3446-3450 (1991).

**Claims**

1. A fibroblast growth factor-2 (FGF-2) antagonist comprising a FGF-2 structural analogue selected from $\text{Gln}^{138}$FGF-2 and $\text{Gln}^{128,138}$FGF-2, wherein said FGF-2 structural analogue exhibits binding affinity to cell surface receptors approximately equal to or greater than native FGF-2, decreased affinity toward heparin or heparin-like polysaccharides compared to native FGF-2, and decreased mitogenic activity compared to native FGF-2.

2. A DNA sequence encoding the FGF-2 structural analogue of claim 1.

3. Use of the FGF-2 antagonist of claim 1 which comprises the $\text{Gln}^{128,138}$FGF-2 structural analogue, for the manufacture of a medicament for the treatment or prophylaxis of angiogenic neoplasms in a mammal.

4. A host cell encoding $\text{Gln}^{138}$FGF-2 deposited under ATCC accession number 69420.

5. A host cell encoding $\text{Gln}^{128,138}$FGF-2 deposited under ATCC accession number 69418.

**Patentansprüche**

1. Fibroblasten-Wachstumsfaktor-2 (FGF-2)-Antagonist, umfassend ein strukturelles FGF-2-Analoges, ausgewählt aus $\text{Gln}^{138}$FGF-2 und $\text{Gln}^{128,138}$FGF-2, worin das strukturelle FGF-2-Analoge eine Bindeaffinität zu Zelloberflächen-Rezeptoren, die etwa gleich oder größer ist als die von natürlichem FGF-2, eine verringerte Affinität zu Heparin oder heparin-artigen Polysacchariden, verglichen mit natürlichem FGF-2, und eine verringerte mytogene Aktivität, verglichen mit natürlichem FGF-2, zeigt.

2. DNA-Sequenz, die für das strukturelle FGF-2-Analoge nach Anspruch 1 codiert.

3. Verwendung des FGF-2-Antagonisten nach Anspruch 1, der das strukturelle $\text{Gln}^{128,138}$FGF-2-Analoge umfasst, für die Herstellung eines Medikamentes zur Behandlung oder Prophylaxe angiogener Neoplasmen in Säugetieren.

4. Wirtszelle, die für $\text{Gln}^{138}$FGF-2 codiert und unter der ATCC-Eingangsnummer 69420 hinterlegt ist.

5. Wirtszelle, die für $\text{Gln}^{128,138}$FGF-2 codiert und unter der ATCC-Eingangsnummer 69418 hinterlegt ist.

**Revendications**

1. Antagoniste du facteur-2 de croissance des fibroblastes (FGF-2) comprenant un analogue structural du FGF-2 sélectionné parmi le $\text{Gln}^{138}$FGF-2 et le $\text{Gln}^{128,138}$FGF-2 où ledit analogue structural de FGF-2 présente une affinité de liaison pour les récepteurs de la surface cellulaire approximativement égale ou supérieure à celle du FGF-2 naturel, une affinité réduite pour l'héparine ou les polysaccharides de type héparine par rapport au FGF-2 naturel et une activité mitogène réduite par rapport au FGF-2 naturel.

2. Séquence d'ADN codant pour l'analogue structural du FGF-2 selon la revendication 1.

**15**

**EP 0 645 451 B1**

3. Utilisation de l'antagoniste du FGF-2 selon la revendication 1 qui comprend l'analogue structural Gln$^{125,138}$FGF-2, pour la fabrication d'un médicament destiné au traitement ou la prophylaxie de néoplasmes angiogéniques dans un mammifère.

4. Cellule hôte codant pour le Gln$^{138}$FGF-2 déposé auprès de l'ATCC sous le numéro de dépôt 69420.

5. Cellule hôte codant pour le Gln$^{128,138}$FGF-2 déposé auprès de l'ATCC sous le numéro de dépôt 69418.

16

Figure 1A

Figure 1B

Figure 2

Figure 3A

Figure 3B

Figure 1

Figure 5